# EUROPEAN PATENT SPECIFICATION

(11) **EP 0 220 916 B1**
(45) Date of publication and mention of the grant of the patent: **27.04.1994**
(21) Application number: 86308150.1
(22) Date of filing: 21.10.1986
(51) Int. Cl.: A61N 1/36, A61N 1/38, A61N 1/39

(54) **Apparatus for recognition of ventricular tachycardia and ventricular fibrillation and for termination thereof**
Gerät zum Erkennen und Beheben von ventrikulären Tachykardien und Fibrillationen
Appareil de détection et de correction de tachycardies et de fibrillations ventriculaires

(30) Priority: 25.10.1985 GB 8526417
(43) Date of publication of application: 06.05.1987
(73) Proprietor: Davies, David Wyn, London E.C.1 (GB)
(72) Inventor: Davies, David Wyn, London E.C.1 (GB)
(74) Representative: Fuchs, Franz-Josef, Dr.-Ing.

(56) References cited:
- EP-A- 0 001 708
- WO-A-82/00415
- US-A- 4 432 375
- IEEE TRANSACTIONS ON BIOMEDICAL ENGINEERING, vol. BME-29, no. 5, May 1982, pages 359-361, IEEE, New York, US; A.R.S. BUKHARI et al.: "Cardiac arrival time analyzer"
- PROCEEDINGS OF THE IEEE, vol. 67, no. 9, September 1979, pages 1322-1337, IEEE, New York, US; L.J. THOMAS Jr. et al.: "Automated cardiac dysrhythmia analysis"

## Description

This invention relates to systems for recognition of ventricular tachycardia and ventricular fibrillation from epicardial electrogram timings and for termination thereof.

Ventricular fibrillation is defined as a condition characterised by fibrillary electrical activity of the ventricular muscle, the electrical impulses traversing the ventricles so rapidly that coordinated contractions cannot occur. This must be distinguished from ventricular tachycardia which may be defined as a rapid (greater than 110 beats per min) cardiac rhythm originating in the ventricles. If sustained, it is usually synchronised in terms of overall ventricular contraction. Both should be differentiated from the normal situation of sinus rhythm where the heart's rhythm is controlled by depolarisation originating from the sinus node and which spread sequentially through the atria, the AV node, the His-Purkinje system and ventricular myocardium.

Apparatus for detecting either of these phenomena is known and indeed WO 82/00415 discloses apparatus for detecting both of these phenomena using two electrodes attached respectively to the superior vena cava and the apical part of the heart and ECG data. Probability data criteria together with heart rate threshold results are used in determining heart rate malfunctioning.

It is an object of the invention to provide a simple system able to recognise both ventricular tachycardia and ventricular fibrillation, whichever may be taking place, and which is to be used with means for responding to both these conditions to restore normal sinus rhythm to the heart.

According to one aspect of the present invention, there is provided an apparatus for automatic recognition of ventricular tachycardia and ventricular fibrillation of a heart and for termination thereof, comprising :
at least a first sensor for attachment to the epicardial surface of one ventricle of a heart and at least a second sensor means for attachment to the epicardial surface of the other ventricle of the heart;
first means for detecting activation sequences derived from signals from the said first and second sensors, with said first means additionally being adapted to determine a time duration between the first and last detected activations in an activation sequence and for determining whether activation sequence and duration are compatible with normal sinus rhythm;
second means for treating ventricular tachycardia;
third means for treating ventricular fibrillation; and
fourth means connected to the first means (a) for identifying and distinguishing between ventricular fibrillation and ventricular tachycardia by detection of change in duration and activation sequence compared to those of a normal sinus rhythm, whereby a change to a different from normal but fixed, i.e. synchrony of activations, activation sequence and an increase of the time duration compared to a time duration of normal rhythm indicates ventricular tachycardia and whereby a loss of synchrony of activations indicates ventricular fibrillation, and (b) for activating said second or said third means according to whether ventricular tachycardia or ventricular fibrillation respectively is indicated.

A preferred form of apparatus embodying the invention is one which additionally comprises third and fourth sensor means for attachment at respective third and fourth epicardial locations of the heart, said detecting means producing third and fourth respective pulse sequences, and said first means being adapted to detect a change in activation sequence from the first to the fourth sensor means.

With one form of apparatus embodying this invention there is included a heart beat detector so that heart beat rate is monitored at all times and activation sequence and duration are determined each time a high heart rate is detected. Such apparatus thus additionally comprises heart beat detector means for determining the time duration between first activations of sequential activations in a series of activations and determining activation sequence when said time duration is detected to be abnormally high.

For a better understanding of the invention and to show how the same can be carried into effect, reference will now be made, by way of example only, to the accompanying drawings wherein:-
FIGURE 1 shows schematically an arrangement of four ventricular activation sites;
FIGURE 2 shows the electrograms obtained from the four sites during normal sinus rhythm;
FIGURES 3A, B and C show electrograms obtained under simulated ventricular tachycardia conditions;

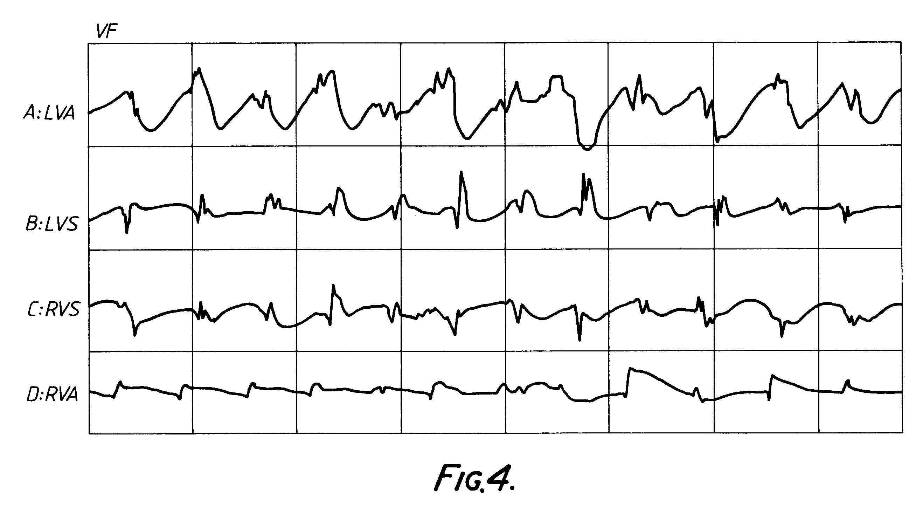

FIGURE 4 shows the electrograms obtained at the four sites under conditions of ventricular fibrillation;
FIGURE 5 is a block diagram of a cardiac implant embodying this invention; and
FIGURE 6 is a flow diagram indicating the characteristic operation of the present invention.

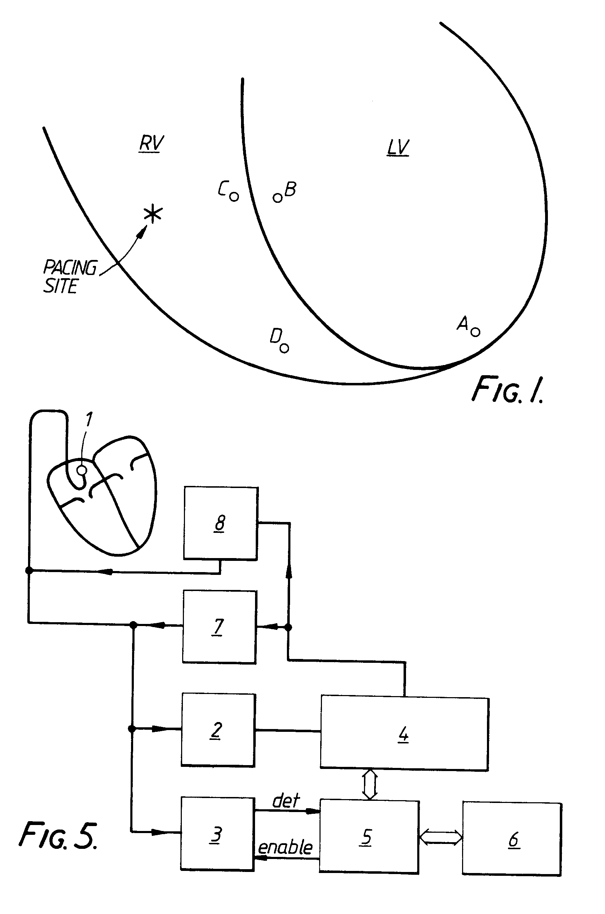

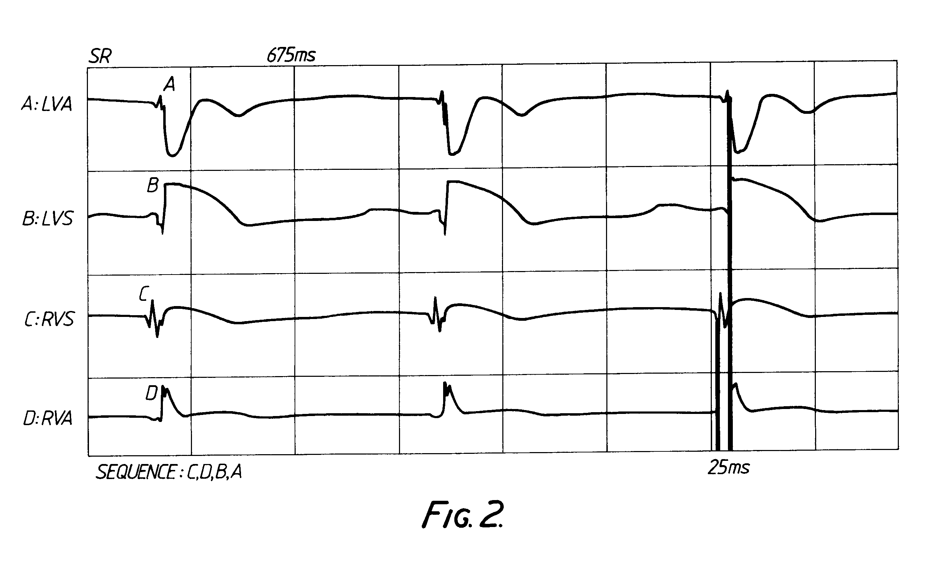

The feasibility of automatic recognition of ventricular tachycardia and ventricular fibrillation has been examined in a number of patients undergoing coronary artery surgery. Bipolar epicardial electrograms from four discrete points on the surface of the heart have been recorded during operation. The points are indicated on the ventricles of the heart. It has been observed that during normal rhythm, the points which are recorded are activated in a certain sequence which is at least consistent, although not always specific to that rhythm. Thus, referring to Figure 1 of the accompanying drawings, the locations of four discrete points numbered A, B, C and D on the left ventricle (LV) and right ventricle (RV) are shown, two of the points (A and D) being on the left ventricular and right ventricular apices and points B and C being at left ventricular and right ventricular paraseptal positions. A pacing site is located on the right ventricle adjacent the third point. During normal rhythm, activation took place in the sequence C, D, B, A in this particular case (see Figure 2). Furthermore, the timing from the first detected deflection to the last of the four was always the same during normal rhythm and in this example, because of normal rhythm, the timing is short, being of the order of 25 msec.

With abnormal rhythm, this timing will generally be increased and the sequence of activations changed.

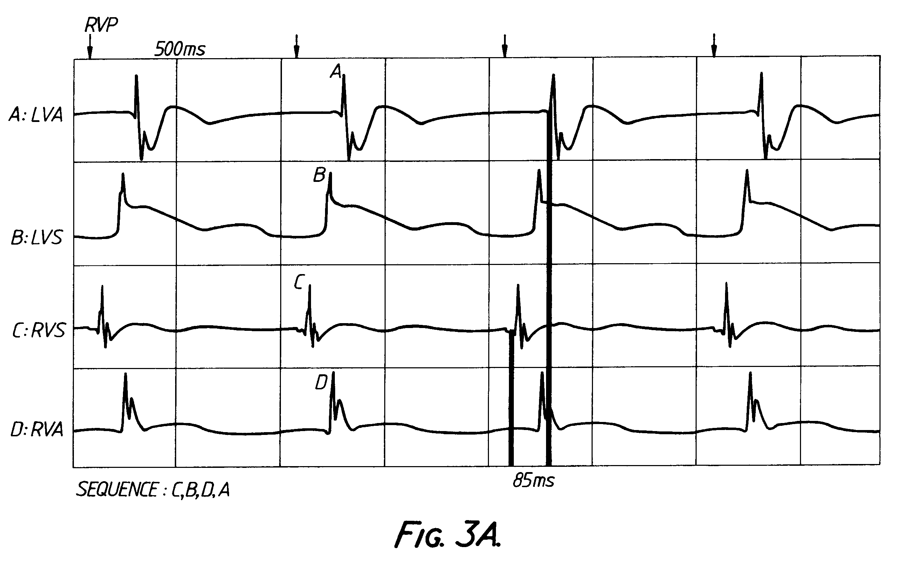

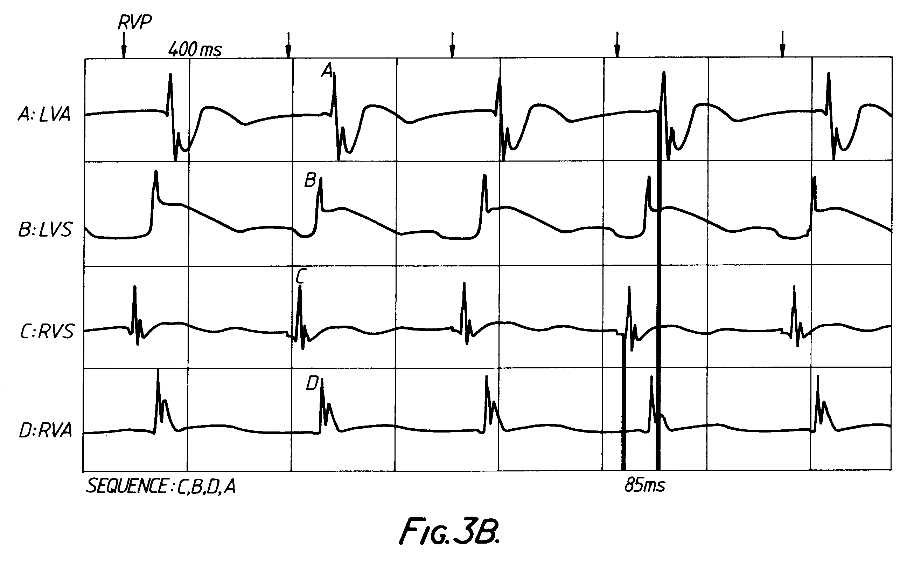

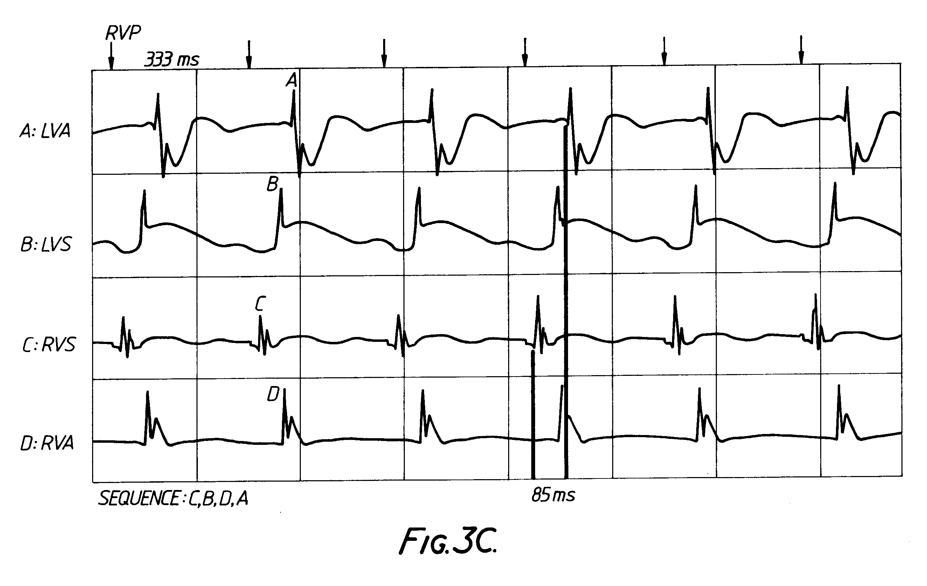

This latter observation was established by simulation of an abnormal rhythm by pacing from the site on the right ventricle. It was observed that 8 out of a group of 10 patients paced at this particular site showed a change of sequence of activation compared with that seen during normal sinus rhythm. Recording of the sequences obtained showed that activations change from C, D, B, A to C, B, D, A. Another abnormality which was induced was that because a normal conducting system was not utilised, the spread of activity took longer across the heart so that the timing from the onset of depolarisation detected first at site C and finally at site A took 85 msecs as opposed to 25 msecs. Figures 3A, 3B and 3C indicate that this duration and sequence of activation is not affected by the rate of the abnormal rhythm provided that its site of origin remains constant.

Maintaining the same set of sites, further experimentation to induce ventricular fibrillation yielded further results of interest. Ventricular fibrillation was induced by putting AC current onto a heart under cardiopulmonary by-pass (this is a means of obtaining cardiac arrest and often used during surgery). It was observed that during ventricular fibrillation, the electrical activity at all four sites was extremely rapid, and certainly more rapid than normally seen. However, there was no apparent fixed sequence of activation. The activity can therefore be described as asynchronous Because of the asynchronous nature of activity, there can be no fixed duration of activity. This thus provides a means of using multi site testing to distinguish between ventricular tachycardia where there is likely to be an altered sequence of depolarisation compared with normal rhythm and an increased duration of activation over that occurring during normal sinus rhythm, and ventricular fibrillation when all this synchrony is lost and the electrical activity from different points in the heart becomes asynchronous

Thus apparatus embodying this invention is programmed to respond to ventricular tachycardia or ventricular fibrillation when they are detected from an altered sequence and duration of ventricular activation as detected by impulses sensed from the epicardial sensing sites.

The present invention is of particular value in that ventricular fibrillation has so far been a very difficult rhythm to detect reliably automatically. Moreover, the energy required by an implantable device to treat ventricular fibrillation is likely to be higher than that required to treat ventricular tachycardia. Therefore by the use of this technique, lower energies can be selected for termination of ventricular tachycardia thereby prolonging battery life. There is thus provided a reliable method for the first time of detecting ventricular fibrillation. The micro-computer utilised in the circuit for comparing activation sequence with that during sinus rhythm can then control a defibrillator which can be discharged when rhythm characteristic of ventricular fibrillation or ventricular tachycardia is detected. Appropriate software is provided for controlling the micro-computer.

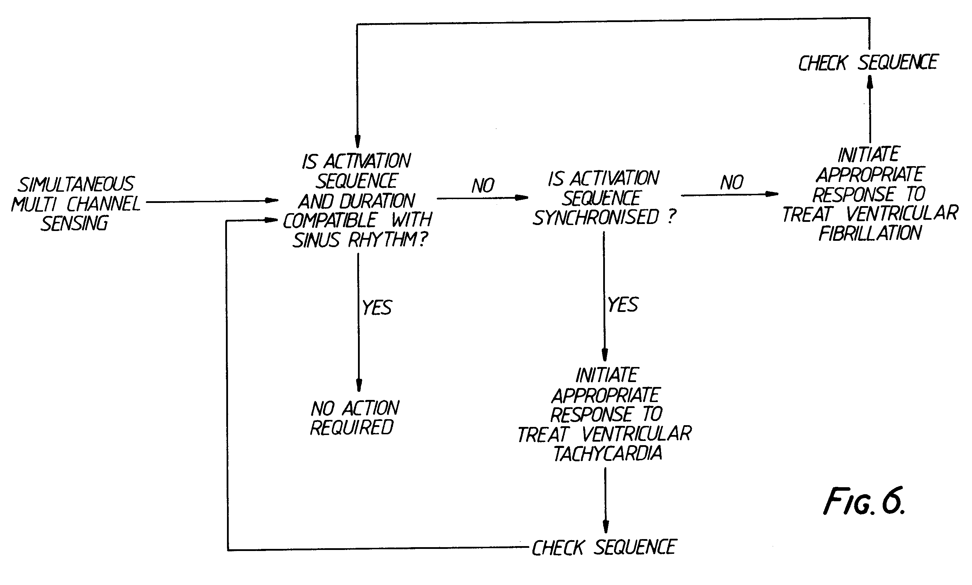

Finally Figures 5 and 6 show practical embodiments of the invention and should be viewed in conjunction with each other. Thus an implant 1 which has sensors (not shown) at positions such as shown in Figure 1) will monitor heart rate beat at all times using a normal heart beat detector 2 having time base and backup pacing control 4 whose operation is directed by a microprocessor 5 having a memory 6. Should a high heart rate be detected, then a detector 3 which is normally operating in backup mode is switched on and simultaneous multi-channel sensing is carried out although Figure 1 shows that sensing at four sites is carried out, and this number is adequate in general, there is no reason why more or less than four sites may be used for testing, although the use of four sites has been found to be an optimum compromise between cost and sensitivity. The microcomputer 5 which is utilised with the detector and receives signals therefrom will check by means of memory 6 whether activation sequence and duration are compatible with sinus rhythm. If this is the case, then no action will be required. However, if the activation sequence and duration are not compatible with sinus rhythm, then provided that an activation sequence is synchronised indicating ventricular tachycardia, a response appropriate to treatment of ventricular tachycardia will be initiated, i.e. stimuli will be delivered by pulse generator 7. In certain cases, which depend on the type of tachycardia, however, ventricular tachycardia may be located by a relatively low energy shock for an associated defibrillator 8. If the activation sequence is not synchronised, indicating that ventricular fibrillation is taking place then operation of the defibrillator 8 will take place.

## Claims

1. An apparatus for automatic recognition of ventricular tachycardia and ventricular fibrillation of a heart and for termination thereof, comprising :
at least a first sensor (A,B) for attachment to the epicardial surface of one ventricle of a heart and at least a second sensor means (C,D) for attachment to the epicardial surface of the other ventricle of the heart;
first means (2,5,6) for detecting activation sequences derived from signals from the said first and second sensors, with said first means additionally being adapted to determine a time duration between the first and last detected activations in an activation sequence and for determining whether activation sequence and duration are compatible with normal sinus rhythm;
second means (7,8) for treating ventricular tachycardia;
third means (8) for treating ventricular fibrillation; and
fourth means (5,6) connected to the first means (a) for identifying and distinguishing between ventricular fibrillation and ventricular tachycardia by detection of change in duration and activation sequence compared to those of a normal sinus rhythm, whereby a change to a different from normal but fixed, i.e. synchrony of activations, activation sequence and an increase of the time duration compared to a time duration of normal rhythm indicates ventricular tachycardia and whereby a loss of synchrony of activations indicates ventricular fibrillation, and (b) for activating said second or said third means according to whether ventricular tachycardia or ventricular fibrillation respectively is indicated.

2. An apparatus according to claim 1, which additionally comprises third and fourth sensor means for attachment at respective third and fourth epicardial locations of the heart, said detecting means producing third and fourth respective pulse sequences, and said first means being adapted to detect a change in activation sequence from the first to the fourth sensor means.

3. An apparatus according to claim 1 or 2, additionally comprising heart beat detector means for determining the time duration between first activations of sequential activations in a series of activations and determining activation sequence when said time duration is detected to be abnormally high.

## Patentansprüche

1. Gerät zum automatischen Erkennen von ventrikulären Tachykardien und ventrikulären Fibrillationen eines Herzens und zum Beenden derselben mit:
mindestens einem ersten Sensor (A,B) zum Anbringen an der epikardialen Oberfläche eines Ventrikels des Herzens und mindestens einem zweiten Sensor (C,D) zum Anbringen an der epikardialen Oberfläche des anderen Ventrikels des Herzens;
ersten Mitteln (2,5,6) zum Detektieren von Aktivitätssequenzen, die von den Signalen des ersten und zweiten Sensors abgeleitet sind, wobei diese ersten Mittel zusätzlich so ausgebildet sind, daß sie die Zeitdauer zwischen der ersten und letzten detektierten Aktivität in einer Aktivitätssequenz bestimmen, und daß sie bestimmen, ob die Aktivitätssequenz und die Zeitdauer mit normalen Sinusrhythmen kompartibel sind;
zweiten Mitteln (7,8) zum Behandeln einer ventrikulären Tachykardie;
dritten Mitteln (8) zum Behandeln einer ventrikulären Fibrillation; und
vierten Mitteln (5,6), die mit den ersten Mitteln (a) zur Identifizierung und Unterscheidung zwischen ventrikulärer Tachykardie und ventrikulärer Fibrillation durch Detektieren einer Änderung der Dauer und der Aktivitätssequenz verglichen mit denen für normale Sinusrhythmen verbunden sind, wobei eine Veränderung zu einer anderen als normalen, aber festen, z.B. synchronisierten Aktivität, Aktivitätssequenz und einem Ansteigen der Zeitdauer verglichen mit der Zeitdauer normaler Sinusrhythmen eine ventrikuläre Tachykardie und der Verlust des Synchronismus der Aktivitäten ventrikuläre Fibrillation anzeigt, und (b) zum Aktivieren der zweiten oder dritten Mittel der je nachdem, ob eine ventrikuläre Tachykardie oder eine ventrikuläre Fibrillation angezeigt wird.

2. Gerät nach Anspruch 1, das zusätzlich dritte und vierte Sensoren zum Anbringen an einer jeweiligen dritten und vierten epikardialen Stelle des Herzens aufweist, bei dem die Mittel zum Detektieren dritte respektive vierte Impulsfrequenzen erzeugen, wobei die ersten Mittel so angepaßt sind, daß sie eine Veränderung in der Aktivitätssequenz vom ersten zum vierten Sensor detektieren.

3. Gerät nach Anspruch 1 oder 2, das zusätzlich einen Herzschlagdetektor zum Bestimmen der Zeitdauer zwischen ersten Aktivitäten von sequenziellen Aktivitäten in einer Folge von Aktivitäten und die Aktivitätssequenz bestimmen, wenn diese Zeitdauer anormal hoch ist.

## Revendications

1. Un appareil destiné à reconnaître automatiquement une tachycardie ventriculaire et une fibrillation ventriculaire d'un coeur et à y mettre fin, comprenant :
au moins un premier capteur (A, B) destiné à être fixé à la surface de l'épicarde d'un ventricule d'un coeur, et au moins un second capteur (C, D) destiné à être fixé à la surface de l'épicarde de l'autre ventricule du coeur ;
des premiers moyens (2, 5, 6) destinés à détecter des séquences d'activation sur la base de signaux provenant des premier et second capteurs, ces premiers moyens étant en outre conçus pour déterminer une durée entre les première et dernière activations détectées dans une séquence d'activations, et à déterminer si la durée et la séquence d'activations sont compatibles avec un rythme de sinus normal ;
des seconds moyens (7, 8) destinés à traiter une tachycardie ventriculaire ;
des troisièmes moyens (8) destinés à traiter une fibrillation ventriculaire ; et
des quatrièmes moyens (5, 6) connectés aux premiers moyens (a) pour identifier et distinguer une fibrillation ventriculaire et une tachycardie ventriculaire, par la détection du changement de durée et de séquence d'activations, en comparaison avec un rythme de sinus normal, grâce à quoi un changement faisant passer à une séquence d'activations différente de la normale mais fixe, c'est-à-dire une condition de synchronisme d'activations, et une augmentation de la durée en comparaison avec une durée de rythme normal, indiquent une tachycardie ventriculaire, tandis qu'une perte de synchronisme d'activations indique une fibrillation ventriculaire, et (b) pour activer les seconds ou troisièmes moyens selon qu'une tachycardie ventriculaire ou une fibrillation ventriculaire est respectivement indiquée.

2. Un appareil selon la revendication 1, comprenant en outre des troisième et quatrième capteurs qui sont destinés à être fixés à des troisième et quatrième emplacements de l'épicarde du coeur, ces moyens de détection produisant respectivement des troisième et quatrième séquences d'impulsions, et les premiers moyens étant conçus de façon à détecter un changement de séquence d'activations, sur la base des signaux que produisent les premier à quatrième capteurs.

3. Un appareil selon la revendication 1 ou 2, comprenant en outre des moyens détecteurs de battement du coeur, qui sont destinés à déterminer la durée entre des premières activations parmi des activations séquentielles dans une série d'activations, et à déterminer la séquence d'activations lorsque la détection de la durée indique que cette dernière est anormalement élevée.
